(19) Europäisches Patentamt
European Patent Office
Office européen des brevets

(11) Publication number: **0 217 491 A1**

# (12) EUROPEAN PATENT APPLICATION

(21) Application number: 86305138.9

(22) Date of filing: 02.07.86

(51) Int.Cl.[4]: C 10 J 3/54
C 07 C 29/15, C 01 C 1/00
C 10 J 3/56

(30) Priority: 28.08.85 US 770140

(43) Date of publication of application: 08.04.87 Bulletin 87/15

(84) Designated Contracting States: DE FR GB IT

(71) Applicant: FOSTER WHEELER USA CORPORATION
110 South Orange Avenue
Livingston New Jersey 07039(US)

(72) Inventor: Froehlich, Robert Edward
24 Brookside Drive
New Providence, New Jersey(US)

(72) Inventor: Zahnstecher, Leonard W.
6 Hastings Lane
Livingston, New Jersey(US)

(74) Representative: Bowman, Paul Alan et al,
LLOYD WISE, TREGEAR & CO. Norman House 105-109 Strand
London WC2R OAE(GB)

## (54) Process for producing ammonia or methanol and a gasifier used in said process.

(57) A process for producing ammonia or methanol and a gasifier used in said process in which the gasifier operates to produce a synthesis gas which is continuously passed to a reactor and a cooler in a closed loop configuration. Ammonia or methanol is produced at the cooler along with a methane rich off-gas. A portion of the latter gas is removed from the loop and is routed back to the gasifier, where the methane therein is converted to produce heat and therefore increase the efficiency of the process.

10
12
14
16
18
19
20
21
22
23
24
25
26
28
30
32
34

FIG. 1

EP 0 217 491 A1

# PROCESS FOR PRODUCING AMMONIA OR METHANOL AND A GASIFIER USED IN SAID PROCESS

## Background of the Invention

This invention relates to a process for producing ammonia or methanol and a gasifier used in said process; and more particularly, to such a process and gasifier in which synthesis gas is produced in the gasifier, and is continuously circulated through a reactor to produce the ammonia or methanol.

Although the most traditional method of utilizing the heat energy of coal is to burn it directly for heat or electric power production, alternative techniques have been proposed in which the coal is converted to more desirable forms of energy such as fuel gas, or liquid hydrocarbons [and] or to chemicals having an enhanced economic value. In the production of fuel or chemicals, the coal is converted in a manner to either remove carbon from, or add hydrogen to, the coal to improve its original hydrogen-to-carbon ratio. One of the more popular techniques of removing carbon is accomplished by gasification.

When coal is completely gasified, the principal components of the gaseous products are hydrogen, carbon monoxide and variable amounts of methane and light hydrocarbons, as well as carbon dioxide. Widely used gas scrubbing techniques are provided for removing the carbon dioxide from the gas, thus removing carbon from the system. Reaction of steam with the coal introduces some hydrogen over that found in the original coal, with the final product being a hydrogen-carbon monoxide gas mixture having any desired hydrogen-to-carbon monoxide ratio. This product, often called "synthesis gas" can be further processed through a reactor, or the like, to form ammonia or methanol, which are relatively clean materials having many applications.

The process of synthesizing gas to produce ammonia or methanol usually involves passing the synthesis gases through a reactor which operates in a conventional manner to combine the carbon monoxide with the hydrogen in the case of methanol, or to combine nitrogen with the hydrogen in the case of ammonia. A cooler, or the like, is usually involved in a closed loop with the reactor, and the gases are continuously circulated through the system with the ammonia or methanol being largely condensed in the cooler and discharged therefrom for further processing. The remaining off-gases from the cooler, which are rich in methane, are recycled back to the reactor and a portion of the methane-rich gas is continuously purged from this loop, since in this type of system the methane is inert. However, the relatively high level of methane production is detrimental to the process efficiency, since it contains valuable hydrogen, and as an inert (instead of a sought after product) it partially offsets the effort to increase the hydrogen to carbon ratio.

## Summary of the Invention

It is therefore an object of the present invention to provide a process for producing ammonia or methanol which enjoys an increased efficiency with respect to prior techniques.

It is a further object of the present invention to provide a process of the above type which utilizes the heat of combustion of the methane to improve the process efficiency.

It is a still further object of the present invention to provide a process of the above type in which the methane from the reactor is rerouted to the gasifier as fuel for the displacement of coal which would otherwise be dedicated for that purpose on a basis of the equivalent BTU value.

It is a still further object of the present invention to provide a gasifier utilized in the above process.

Toward the fulfillment of these and other objects, the present invention features the provision of a fluidized bed gasifier which operates to produce a synthesis gas which is continuously passed to a reactor and a cooler in a closed loop configuration. Ammonia or methanol is produced at the cooler along with a methane rich off-gas. A portion of the latter gas is removed from the loop and is routed back to the gasifier, where the methane therein is converted to produce heat and therefore increase the efficiency of the process.

## Brief Description of the Drawings

The above brief description as well as further objects, features and advantages of the present invention will be more fully appreciated by reference to the following detailed description of presently preferred but nonetheless illustrative embodiments in accordance with the present invention when taken in conjunction with the accompanying drawings wherein:

Fig. 1 is a schematic view depicting a system utilizing the present invention; and

Fig. 2 is a somewhat diagrammatic cross-sectional elevational view of a gasifier used in the process of the present invention.

## Description of the Preferred Embodiment

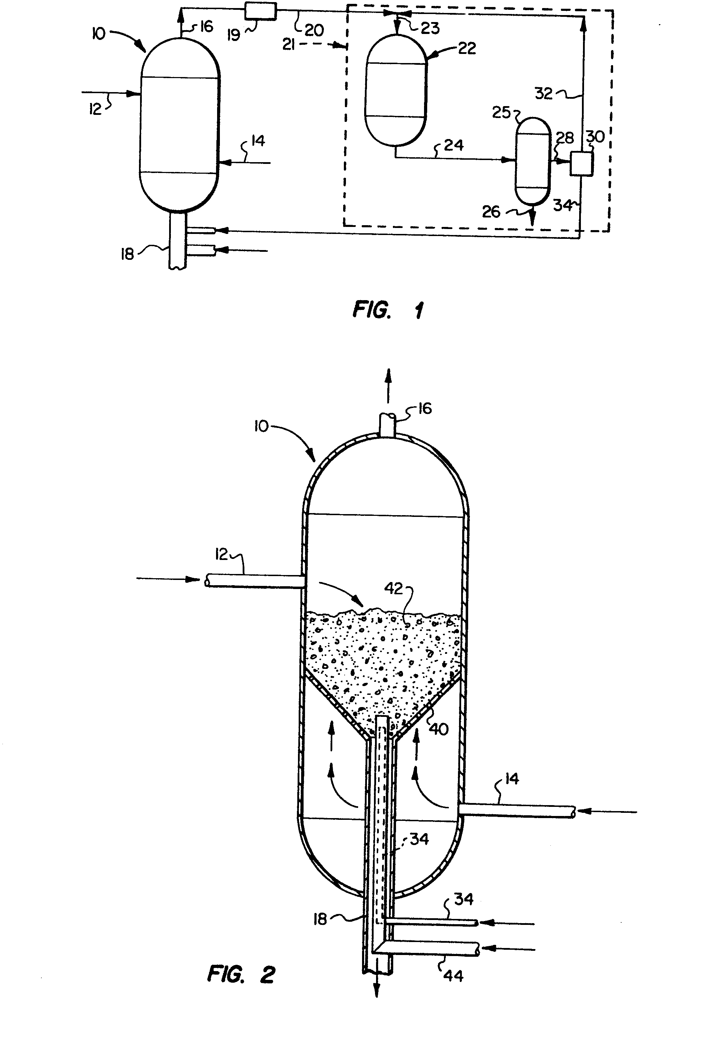

The present invention will be described, for the purpose of example, in connection with a system for producing ammonia which is shown in Fig. 1 of the drawing. The reference numeral 10 refers, in general, to a fluidized bed gasifier which receives particulate coal from a conduit 12 and air from a conduit 14. A conduit 16 extends from the upper end of the gasifier for discharging the synthesis gases formed in the gasifier, and a drain pipe 18 extends from the lower end of the gasifier for discharging spent coal, preferably as agglomerated ash. Further details of the gasifier will be described later.

The synthesis gases formed in the gasifier 10, which contain essentially hydrogen, nitrogen and methane as well as carbon monoxide, carbon dioxide, steam and hydrogen sulfide, are then passed to a pretreatment station 19 for shifting the carbon monoxide and the steam in the synthesis gases to form carbon dioxide and hydrogen, for removing the carbon dioxide and the hydrogen sulfides from the gases and for performing a final sulfur clean-up as needed. Since these techniques are conventional they will not be described in any further detail. The outlet of the pretreatment station 19 is connected, via a conduit 20, to a processing unit, shown in general by the reference numeral 21, and extending within the dashed lines. The processing unit 21 is for the purpose of further processing the gases from the gasifier 10 in a conventional manner to produce ammonia.

The processing unit 21 includes a reactor 22 which has an inlet conduit 23 for receiving the gases from the gasifier 10. The reactor 22 operates in a conventional manner, and, as such, contains a catalyst to promote the combination of the nitrogen and hydrogen from the latter gases. The off gases from the reactor 22 are discharged, via a conduit 24, to a cooler 25.

The cooler 25 operates in a conventional manner to cool the gases from the reactor 22 to the extent that liquid ammonia is formed, it being understood that auto refrigeration can be used in the cooler to the extent necessary. The ammonia is discharged, via a conduit 26, to external equipment (not shown) for other uses.

The remaining gases in the cooler 25 are recycled, via a conduit 28, a purge unit 30 and a conduit 32, back to the inlet conduit 23 for reintroduction into the reactor 22. The purge unit 30 operates in a conventional manner to vent, or purge, a small portion (for example, 5%) of the gases received from the conduit 28, which gases contain a relatively high ampunt of methane which is an inert gas in the foregoing process. Since this technique of continuously circulating the synthesis gases received from the gasifier 10 through the processing unit 21 while producing the product gas, in this case ammonia, and purging methane, is conventional, it will not be described in any further detail.

According to a main feature of the present invention, the purged methane-rich gas from the purge unit 30 is routed, via a conduit 34, to the interior of the drain pipe 18 of the gasifier 10, for reasons that will be described.

As shown in Fig. 2, the drain pipe 18 extends from the apex of a downwardly oriented frusto-conically shaped, perforated, air distributor plate 40 disposed within the housing of the gasifier 10, with the lower end portion of the pipe projecting from the lower end of the gasifier housing. A bed 42 of particulate coal, supplied by the conduit 12, is supported by the plate 40, and the drain pipe 18 serves to discharge ashes and spent coal from the bed to external equipment (not shown).

A conduit 44 supplies air and steam from external sources, and includes a horizontal portion that extends through an appropriate opening formed in the drain pipe 18, and a vertical portion that extends upwardly in a coaxial relationship with the drain pipe. The outer diameter of the conduit 44 is less than the inner diameter of the drain pipe 18 to form an annular passage for the spent coal and ashes discharged from the bed 42.

The conduit 34 includes a horizontal portion extending from the processing unit 21 and through appropriate openings in the drain pipe 18 and the conduit 44, and a vertical portion extending upwardly within the conduit 44 in a coaxial relationship. The outer diameter of the conduit 34 is less than the inner diameter of the conduit 44 to provide a passage for the air and steam through the latter conduit.

The respective end portions of the conduits 34 and 44 extend through an opening at the apex of the air distributor plate and slightly into the lower portion of the bed 42. Thus, methane-rich gas through from the processing unit 21 along with air and steam, are introduced into the bed 42 via the conduits 34 and 44, respectively.

The air conduit 14 extends through a side wall of the housing of the gasifier 10 for supplying air from an external source at a relatively high pressure into the gasifier. This pressurized air passes upwardly through the perforations in the plate 40 and into the bed 42 to fluidize the particulate coal in the bed and promote the combustion thereof.

In operation, controlled quantities of pressurized air are introduced into the conduit 14, and pass upwardly through the perforated plate 40 and into the bed 42. Concurrently, controlled quantities of pressurized air and steam, are introduced through conduit 44. The velocity of the air thus introduced is controlled to fluidize the particulate coal in the bed 42 and promote the combustion of the coal. The quantity of air is controlled so that it is insufficient for complete combustion of the coal but sufficient to produce a synthesis gas including hydrogen, carbon monoxide, carbon dioxide and small quantities of methane. It is understood that a bed light off burner, or the like (not shown) is provided for initially lighting off the bed during start-up, and additional coal is added to the bed via the conduit 12.

The synthesis gases thus produced in the gasifier 10 along with the steam or water, are passed to the pretreatment station 19 for the treatment described above, to produce hydrogen, nitrogen and methane. The gases then pass through the inlet conduit 23 and into the reactor 22, it being understood that the temperature and other critical conditions of the reactor 22 are maintained in a predetermined manner, so as to cause the hydrogen to combine with the nitrogen, with the resulting gas being discharged, along with the remaining gases, from the reactor 22. The gases then pass, via the conduit 24, to the cooler 25.

The cooler 25 operates to form the ammonia in the manner described above which is discharged to external equipment (not shown) via the discharge conduit 26. The remaining off-gases exit the cooler 25 through the conduit 28 and pass to the purge unit 30, which operates to vent, or purge, a small portion of the gases to the conduit 34. The remaining gases are discharged from the purge unit 30 to the conduit 32 and pass back into the inlet conduit 23 for recycle back to the reactor 22. Thus a continuous loop is formed consisting of the reactor 22, the cooler 25 and the purge unit 30.

The methane-rich gas from the conduit 34 is introduced coaxially through the drain pipe 18 and into the fluidized bed 42, whereby the oxygen from the air in the bed converts the methane to carbon monoxide and hydrogen, which blend with the constituents of the synthesis gas formed in the gasifier and are discharged to the conduit 16. The conversion of the methane in the gasifier 10 produces a heat of combustion which lowers the coal requirements in the gasifier.

It is thus seen that the process and apparatus of the present invention enjoys an increased efficiency with respect to prior techniques by utilizing the heat of combustion of the methane to improve the process efficiency.

It is understood that the process of the present application has equal applicability to the production of methanol or of any chemical which can be formed in the above manner with the production of a methane-rich gas. Thus, processing unit 21 may be alternatively viewed as a methanol plant, in which methanol-forming reactions are performed in reactor 22, and in which cooler 25 produces an off-gas which is relatively methane-rich. In accordance with the invention, the off-gas stream is split, with a portion returned to the reactor for further conversion to methanol, another portion returned to the gasifier 10, and a third portion may be vented through a vent (not shown) or otherwise disposed of.

Other modifications, changes and substitutions are intended in the foregoing disclosure and in some instances some features of the invention will be employed without a corresponding use of other features. Accordingly, it is appropriate that the appended claims be construed broadly and in a manner consistent with the spirit and scope of the invention therein.

What is claimed is:

1. A process for producing ammonia or methanol comprising the steps of providing a fluidized bed of particulate coal, passing an oxidant gas through said coal in sufficient quantities and at a sufficient velocity to fluidize and promote the combustion of said particulate coal, controlling the temperature of, and the air or oxygen input to, said bed to gasify said coal to produce a synthesis gas, processing said synthesis gas in a manner to produce ammonia or methanol and an off gas rich in methane, removing a portion of said off-gas, and passing said removed off-gas to said fluidized bed to react with said air to convert the methane in said off-gas and thereby replace a portion of said coal dedicated to fuel use in said coal bed.

2. The process of claim 1 further comprising the step of adding steam to said gasifier.

3. The process of claim 1 wherein said synthesis gas contains hydrogen, carbon monoxide, nitrogen and steam; and further comprising the steps of shifting said carbon monoxide and steam to carbon dioxide and hydrogen, and removing said carbon dioxide from said synthesis gas.

4. The process of claim 3 wherein said methane is converted to carbon monoxide and hydrogen which blend with the carbon monoxide and hydrogen from said synthesis gas.

5. The process of claim 3 wherein said step of processing comprises the step of combining said hydrogen with said nitrogen to produce ammonia.

6. The process of claim 4 wherein said step of processing further comprises the step of circulating said gases through a reactor for performing said step of combining and through a cooler for forming said methanol or ammonia and said off-gas.

7. The process of claim 6 wherein said step of processing further comprises continuously circulating the remaining portion of said off-gas back through said reactor and said cooler.

8. A fluidized bed gasifier comprising a vessel, a perforated plate disposed in said vessel for supporting a fluidized bed of particulate fuel material, means for introducing an oxidant gas into said bed to fluidize and promote the combustion of said fuel material, and means for introducing methane into said bed which reacts with said oxygen to convert said methane to produce heat.

9. The gasifier of claim 8 further comprising a drain pipe registering with an opening in said perforated plate for permitting the spent fuel to discharge from said bed, said methane introducing means comprises a pipe extending through said drain pipe and having a projecting portion extending into said bed.

10. The gasifier of claim 9 wherein said air introducing means comprise a pipe extending through said drain pipe and having a projecting portion extending into said bed.

11. The gasifier of claim 10 wherein said air introducing means further comprises means for introducing air into said vessel below said perforated plate for passing through said perforations and into said plate.

10
12
14
16
18
19
20
21
22
23
24
25
26
28
30
32
34


*FIG. 1*

10
12
14
16
18
34
34
40
42
44


*FIG. 2*

European Patent Office

# EUROPEAN SEARCH REPORT

0217491

Application number

EP 86 30 5138

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | EP-A-0 078 591 (EXXON)<br>* Pages 25,26, claim 1 * | 1,2 | C 10 J 3/54<br>C 07 C 29/15<br>C 01 C 1/00<br>C 10 J 3/56 |
| A | | 4,6,7 | |
| Y | US-A-2 713 590 (PALMER)<br>* Column 6, lines 3-48 * | 1,2 | |
| A | EP-A-0 126 961 (WESTINGHOUSE)<br>* Page 3, line 7 - page 4, line 11 * | 3-5 | |
| A | EP-A-0 027 280 (INSTITUTE OF GAS TECHNOLOGY)<br>* Page 8, line 30 - page 10, line 25 * | 8-11 | **TECHNICAL FIELDS SEARCHED (Int Cl.4)** |
| A | FR-A- 843 515 (METALLGESELLSCHAFT)<br>* Pages 3,4, abstract * | 1 | C 10 J |
| X | EP-A-0 130 846 (FOSTER WHEELER)<br>* Page 4, line 8 - page 6, line 6; claim 1 * | 1-7 | |

The present search report has been drawn up for all claims

| Place of search | Date of completion of the search | Examiner |
|---|---|---|
| THE HAGUE | 09-12-1986 | WENDLING J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82